# EUROPEAN PATENT APPLICATION

(11) **EP 1 607 109 A1**
(43) Date of publication of application: **21.12.2005**
(21) Application number: 05397012.5
(22) Date of filing: 14.06.2005
(51) Int. Cl.: A61L 27/48, A61L 27/54, A61L 31/12, A61P 19/00, A61P 29/00

(54) **Multifunctional biodegradable composite and surgical implant comprising said composite**

(30) Priority: 15.06.2004 FI 20045223
(71) Applicant: BIORETEC OY, 33720 Tampere (FI)
(72) Inventor: Ashammakhi, Nureddin, 33100 Tampere (FI); Suokas, Esa, Tampere, 33580 (FI); Törmälä, Pertti, Tampere, 33300 (FI); Viitanen, Petrus, 33100, Tampere (FI)
(74) Representative: Hakola, Unto Tapani

(57) **Abstract**

A multifunctional biodegradable composite has:
a) bioabsorbable polymer matrix phase (M)
b) bioabsorbable reinforcing element (R), and
c) bioactive tissue/cell reaction modifying agent (TRMA) dispersed in said bioabsorbable matrix phase and selected from the group consisting of anti-inflammatory drugs and statins.

Said biodegradable composite may be in a surgical implant capable of acting as a drug-delivery implant.

## Description

The present invention relates to a multifunctional biodegradable composite and a surgical implant comprising said composite.

Biodegradable composites comprising biodegradable matrix phase and biodegradable reinforcing element are well known in the field of surgical devices. Such composites may also contain bioactive agents for controlled release into the body where the device has been implanted. Examples of documents describing such composites include US 6,406,498 and EP 1233794.

As disclosed in U.S. Patent 6,685,928, (published on Aug. 8, 2002 as application 2002/0106345) it has been discovered that the local administration of an anti-inflammatory agent to tissue provides beneficial effects on the healing and growth of the tissue and on proximally located tissues. The patent provides a method to promote healing of hard tissue comprising administering an effective amount of an anti-inflammatory agent to the hard tissue or to soft tissue near the hard tissue. The patent also provides a method of treating periodontal disease comprising administering an effective amount of an anti-inflammatory agent at the site of the periodontal disease. The patent also provides a method of treating a bone fracture comprising fixing the fracture with an orthopedic device comprising an anti-inflammatory agent in the polymeric backbone of an aromatic polyanhydride. The patent also provides a method to enhance regeneration of hard tissue comprising administering an effective amount of an anti-inflammatory agent to the hard tissue or to soft tissue near the hard tissue. It also provides a method to decrease bone resorption at a site in the body of a patient comprising administering an effective amount of an anti-inflammatory agent at or near the site. The preparation of aromatic polyanhydrides from ortho-substituted bis-aromatic carboxylic acid anhydrides disrupts the crystallinity of the resulting polymer, enhancing solubility and processability, as well as degradation properties. The use of hydrolyzable bonds such as esters, amides, urethanes, carbamates and carbonates as opposed to aliphatic bonds in these compounds further enhances these properties. The hydrolysis products of the polyanhydrides have the chemical structure of an anti-inflammatory agent, particularly salicylates such as aspirin, non-steroidal anti-inflammatory compounds, or other aromatic anti-inflammatory compounds.

The aromatic polyanhydrides of the invention in the said patent meet the need for moldable biocompatible biodegradable polymers and are particularly useful in enhancing the healing process of bone and surrounding soft tissue.

The said US Pat 6,685,928 relates to compositions and methods of using compositions comprising aromatic polyanhydride with a repeating unit of a certain formula to enhance healing of tissue (e.g. hard tissue). It has been found that these compositions promote healing in hard tissue by inhibiting inflammation and/or pain in the surrounding soft tissues and by enhancing hard tissue regeneration by promoting growth and/or by reducing bone resorption. To use these compositions to enhance tissue regeneration, it is preferred that the compositions be incorporated into fibers, films, membranes, pastes or microspheres. For this use, it is also preferred that the compositions comprise poly(anhydride-esters), referred to herein as bioactive polyanhydrides that degrade into salicylic acid, an anti-inflammatory, antipyretic and analgesic agent. The hard tissue and surrounding soft tissue are directly contacted with the composition so that regeneration and healing is enhanced.

US Pat 5,711,958 discloses a method for reducing or eliminating post-surgical adhesion formation by affixing a polymeric composition comprising a block copolymer of ABA triblock type in a patient's body. The polymeric composition can be in a film, viscous solution or gel form. The polymer can also be used to deliver bioactive agents to a site of activity within the patient's body, one example of the listed agents being steroidal and non-steroidal anti-inflammatory agents. It is mentioned in the patent that the polyester A blocks of the triblocks of the polymer tend to be biodegradable, whereas the poly(oxyalkylene) B blocks of the triblocks and chain extenders tend not to be biodegradable. It is recognized in the patent (column 11, lines 40 - 43) that the poly(oxyalkylene) B blocks will remain as polymeric units in vivo until such time as the blocks are excreted. The patent is silent about the form and structure of the polymeric composition that would be suitable for delivering the bioactive agent to the body, as well as about the way of incorporating the agent in the polymeric composition. In the practice, the above-mentioned polymer is found to be too slowly biodegradable.

It is an object of the present invention to provide a composite where the release properties of a bioactive, tissue/cell reaction modifying agent are adjustable to follow a desired pattern, and combined with some favourable mechanical strength properties.

The object of the present invention are obtained by the combination of the bioactive agent an anti-inflammatory agent and biodegradable polymer matrix which is self-reinforced and contains dispersed phase of said agent, which is selected from a group consisting of tissue-reaction modifying agents such as anti-inflammatiory agents and statins.

### BRIEF DESCRIPTION OF THE DRAWINGS

- FIG. 1: illustrates a schematic sectional view of the structure of a multifunctional, tissue reaction modifying agent releasing implant as constructed in accordance with one embodiment.
- FIG.2: illustrates a schematic sectional view of the structure of a multifunctional, tissue reaction modifying agent releasing implant as constructed in accordance with another, advanced embodiment,
- FIG. 3: is a schematic figure of the arrangement of a solid-state die drawing process as one example of the reinforcing technique,
- FIG. 4: shows the daily release of a tissue-reaction modifying agent from various multifunctional implants, and
- FIG. 5: is a SEM-image of a structure of the implant containing particles of a tissue-reaction modifying agent

### DETAILED DESCRIPTION OF THE INVENTION

Local administration of an anti-inflammatory agent or statin on or near hard tissue, such as bone or tooth, enhances the growth and regeneration of the hard tissue and the surrounding soft tissue. Preferably, the anti-inflammatory agent or the statin is administered in a form that provides a controlled release of the agent at or near the hard tissue over a period of days or months.

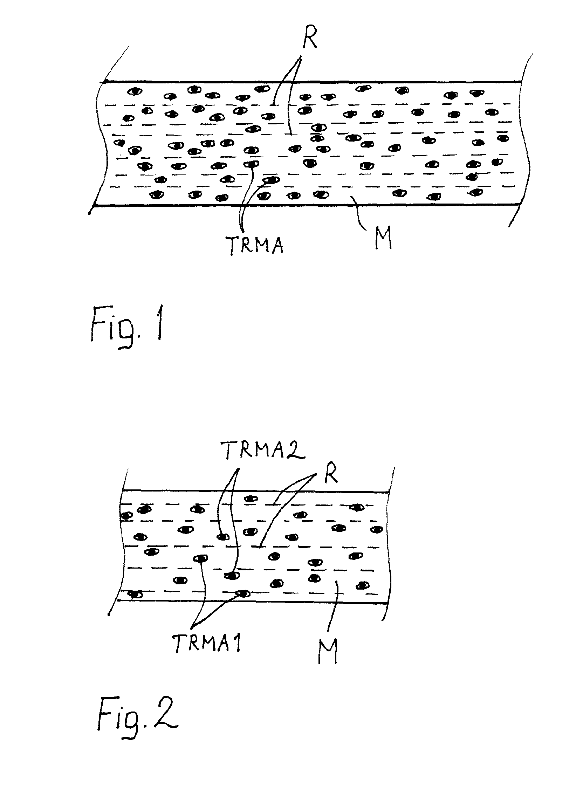

The composite comprises three main constituents, which are shown schematically in Fig. 1, namely 1) a matrix phase M consisting of a biodegradable synthetic polymer (or a blend of such polymers), 2) a biogegradable reinforcing element R which may also be a biodegradable synthetic polymer, for example of the same composition as the matrix due to orientation/self-reinforcing technique of the matrix, and 3) a bioactive agent TRMA dispersed in said matrix. The agent is a tissue/cell reaction modifying agent, the alterantives of which are described more closely below. Optionally, a fourth additive to assist osteoconduction, osteoinduction, handling, visualization, or to provide additional synergistic therapeutic effect may be included in the matrix M. Typical examples of the last group additives are different bioceramics such as calcium phosphates that show osteoconductive effect in different indications.

### Anti-inflammatory drugs

Anti-inflammatory drugs can be classified into steroidal and non-steroidal anti-inflammatory drugs (NSAID). Steroids are potent anti-inflammatory and immunosuppressive drugs. Problems with steroids are their side effects such as hyperacidity, development of peptic ulcer, appearance of oedema and development of hypertension and diabetes [Kapoor O. "Side effects of NSAID type of Drugs as against SAID (steroids)" [http://www.bhj.org/journal/2002 4404 oct/gps 670.htm] Accessed on 7. November 2003].

Anti-inflammatory agents particularly useful in the methods of the invention include non-steroidal anti-Inflammatory drugs (NSAIDs). NSAIDs typically inhibit the body's ability to synthesize prostaglandins. Prostaglandins, important mediators of inflammation, are a family of hormone-like chemicals, some of which are made in response to cell injury. Inhibiting prostaglandin production results in analgesic, antipyretic and anti-inflammatory effects. Specific NSAIDs approved for administration to humans include naproxen sodium, diclofenac, sulindac, oxaprozin, diflunisal, aspirin, piroxicam, indomethocin, etodolac, ibuprofen, fenoprofen, ketoprofen, mefenamic acid, nabumetone, tolmetin sodium, and ketorolac tromethamine.

NSAIDs are among the most frequently used drugs world-wide. About seven million prescriptions of NSAIDs are written in USA every year. NSAIDs are useful for the treatment of mild to moderate pain [McGrath P.J.. Rice A.S.C. Pain - Basic Science. http://www.centef.ch/pain/. © OPAL Open Programs for Associative Learning SA 1997-1999. © Bahram Zaerpour 1999]. They act by inhibiting the activation of free nerve endings by inhibition of the prostaglandin (PG) synthesis.

Other anti-inflammatory agents useful in the methods of the invention include salicylates, such as, for example, salicilic acid, acetyl salicylic acid, choline salicylate, magnesium salicylate, sodium salicylate, olsalazine, and salsalate.

Other anti-inflammatory agents useful in the methods of the invention include cyclooxygenase (COX) inhibitors. COX catalyzes the conversion of arachidonate to prostaglandin H2 (PGH2); a COX inhibitor inhibits this reaction. COX is also known as prostaglandin H synthase, or PGH synthase. Two Cox genes, Cox-1 and Cox-2 have been isolated in several species. COX-2 is tightly regulated in most tissues and usually only induced in abnormal conditions, such as inflammation, rheumatic and osteo-arthritis, kidney disease and osteoporosis. COX-1 is believed to be constitutively expressed so as to maintain platelet and kidney function and integral homeostasis. Typical COX inhibitors useful in the methods of the invention include etodolac, celebrex, meloxicam, piroxicam, nimesulide, nabumetone, and rofecoxib.

### Statins

Statin drugs are currently the most therapeutically effective drugs available for reducing the level of low-density lipoproteins (LDL) in the blood stream of a patient at risk for cardiovascular disease. They function by limiting cholesterol biosynthesis by inhibiting the enzyme HMG--CoA reductase. They include lovastatin, pravastatin, simvastatin, compactin (mevastatin), atorvastatin, fluvastatin, and cerivastatin. All these statin drugs share a common mechanism of action and have similar toxicity profiles.

For example, simvastatin is the common medicinal name of the chemical compound butanoicacid, 2,2-dimethyl-,1,2,3,7,8,8a-hexahydro-3,7-dimethyl-8-[2-(tetrahydro-4-hydroxy-6-oxo-2H-pyran-2-yl)-ethyl]-1-naphthalenyl ester, [1S*-[1a,3a,7b,8b(2S*,4S),-8ab]]. (CAS Registry No. 79902-63-9.).

Lovastatin is the common medical name of the chemical compound [1S-[1.alpha.(R*),3.alpha.,7.beta.;8.beta.(2S*,4S*),8a.beta.]]-1,2,3,7,8,8a-hexahydro-3,7-dimethyl-8-[2-(tetrahydro-4-hydroxy-6-oxo-2H-pyran-2-yl ethyl]-1-naphthalenyl 2-methylbutanoate. (CAS Registry No. 75330-75-5.)

Statin compounds are also known to have the metabolic effect of directly enhancing bone growth (US Patent 6,376,476). Use of at least one compund classified as statin by virtue of its ability to inhibit HMG-CoA reductase will help to stimulate the growth of bone tissue when this bioactive agent is released from the composite implanted in the body. According to the current knowledge, simvastatin is preferred among statins in the present invention, because in clinical research it has proved advantageous on account of BMP-2.

### Bioabsorbable polymers

By bioabsorbable polymer is understood a synthetic or naturally derived bioabsorbable, biocompatible polymer that is absorbable (resorbable) in tissue conditions, i.e. once implanted in a living mammalian body. Synthetic polymers can include poly-α-hydroxy acids (e.g. polylactides, polycaprolactones, polyglycolides and their copolymers, such as lactide / glycolide copolymers and lactide /caprolactone copolymers), polyanhydrides, polyorthoesters, polydioxanone, segmented block copolymers of polyethylene glycol and polybutylene terephtalate (Polyactive™), poly(trimethylenecarbonate) copolymers, tyrosine derivative polymers, such as tyrosine-derived polycarbonates, poly(ester-amides), polyamides, polyoxalates, polyacetals, polyiminocarbonates, polyurethanes, polyphosphonates or injectable polymers such as polypropylenefumarates. This last polymer group can be cross-linked after injecting onto surface of a reinbforced or self-reinforced product to form a thin layer. This layer can include bioactive agents or they can be absentSuitable bioabsorbable polymers to be used as matrix materials in manufacturing of composites of the present invention are mentioned e.g. in U.S. Pat. Nos. 4,968,317, 5,618,563, FI Patent No. 98136, FI Patent No. 100217B, and in "Biomedical Polymers" edited by S. W. Shalaby, Carl Hanser Verlag, Munich, Vienna, New York, 1994 and in many references cited in the above publications.

A polymer listed above can form the matrix phase alone. However, the term bioabsorbable polymer matrix phase shall be understood to mean also a matrix comprising a blend of two or several different bioabsorbable polymers that differ from each other physically and/or in chemical structure. Matrix material can be thermoplastic such as polyalfa-hydroxy acids or thermosets such as polypropylenefumarates. The later polymer group is suitable only as a coating for reinforced or self-reinforced structures.

The above list is not meant to be exhaustive.

### Reinforcing element

The matrix polymer (or blend of matrix polymers) is in close association with a bioabsorbable or bioactive reinforcing element that contributes to the strength or other desired functions of the implant device, which is an important factor if the composite is intended for use in bone fixation and other similar applications. A special case is the function of the matrix both as the carrier material and reinforcing structure, e.g. using self-reinforcing technique during the manufacture of the composite. Such techniques are based on mechanical modification of the bioabsorbable polymer raw material, and may include orientation and fibrillation of partly crystalline materials according to U.S. Patent 4,968,317, or US 6,406,498 (self-reinforcement). In this case the reinforcing element also possesses bioabsorbable properties.

Another alternative is creating discrete areas of matrix and reinforcing structure in the implant device, and at least the matrix contains at the same time the bioactive agent in the form of an anti-inflammatory drug or a statin. The reinforcing structure may be of the same chemical composition as the matrix and be embedded in the same. An example of such a composite structure is disclosed e.g. in U.S.Patent No. 4,743,257. This structure is also termed "self-reinforced" because of the common origin of both matrix and the reinforcing structure. The reinforcing element is bioabsorbable also in this case.

The discrete areas of matrix and reinforcing structure can be composed of chemically different polymers, both being biocompatible and bioabsorbable (bioresorbable), and the polymer of the reinforcing structure being selected because of its mechanical properties (strength). This is suitable for matrix polymers that are nor eligible for self-reinforcing techiques.

It is also possible that the reinforcing element can be bioabsorbable inorganic materials, for example in the form of fibers of bioabsorbable bioactive glass, as described in U.S.Patent No. 6,406,498, or in the form of any other material that acts as a reinforcing structure for the matrix and is at the same time bioabsorbable. This can also be used for matrix polymers that are not eligible for self-reinforcing techiques

The parts designated "R" in Fig. 1 are meant to cover all the above-mentioned alternatives of the reinforcing element, and it should be noted that the representation of Fig. 1 is meant to serve as a simplified illustration of the structure and not as an exact reproduction of it.

As was mentioned above, the anti-inflammatory drug or the statin forms a dispersed phase in the matrix. This bioactive agent (TRMA in Fig. 1) is in the form of discrete particles or particle clusters. If a self-reinforcing technique by mechanical modification of the matrix polymer is used, it is also possible to create voids in the matrix around the particles or particle clusters (also shown schematically in Fig. 1), which are advantageous in adjusting the release profile. Furthermore, the voids improve the mechanical properties by increasing the flexibility. The voids are typically elongated in the direction of orientation.

It is also possible that the particles or particle clusters of the bioactive agent TRMA are distributed in the matrix without voids, in the case where the matric is reinforced with a reinforcing element not originating in the matrix polymer.

It is also possible that the disperse phase in the matrix, in addition to the bioactive agent, contains some inorganic particles, for example bioactive glass particles or other bioactive ceramic particles, especially when the implant is to be used for bone repair (e.g. bone fixation), bone augmentation, bone regeneration (e.g. guided bone regeneration), or similar applications.

It is also possible that the dispersed phase in the matrix comprises both an anti-inflammatory drug and a statin for dual tissue reaction modifying function of the composite. This alternative is shown in Fig. 2, where the same signs are used as in Fig. 1, except that the first bioactive agent is denoted with "TRMA1", and the second agent of another kind with "TRMA2". As can be seen, voids also exist in connection with the particles of these two tissue reaction modifying agents TRMA1, TRMA2 as a result of the self-reinforcement by orientation.

Finally, it is possible that in the combination matrix/reinforcing element/TRMA an additional fourth component of biological activity is added. The fourth component can also form a disperse phase in the matrix and it can be a bioceramic particle or fibre, such as calcium phosphate or bioactive glass.

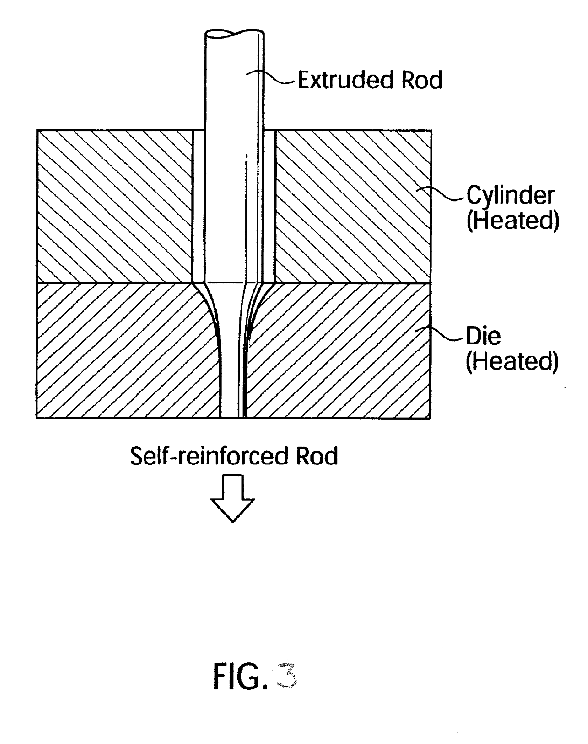

Fig. 3 illustrates the principle of self-reinforcement based on solid-state mechanical modification of a preform containing the polymer and the bioactive agent dispersed therein, created by compounding the polymer and the bioactive agent by a suitable melt-processing techique. Fig. 3 illustrates a die-drawing process, where the solid-state preform is forced through a die that effects a predetermined draw-ratio. Other drawing techiques for achieving the orientation can also be employed. The examples of such techniques as hydrostatic extrusion and free drawing method like fiber spinning together with subsequent sintering process. The orientation is typically accomplished at a temperature above the T_{g} (glass transition temperature) of the matrix polymer, when the polymer is amorphous, but below its melting temperature,, when the polymer is semicrystalline or crystalline). Even though a cylindrical rod is shown as an example of the preform, the preform can be also of another shape, for example plate-like, and the die is shaped and dimensioned correspondingly, if die-drawing is used as the drawing technique for the preforms of other shapes. Possible performs includes filaments, films, tubes or profiles.

The draw ratio can be between 2 and 12, depending on the properties of the polymer, typically 3 or higher.

The invention is described in the following examples which do not restrict the scope of the invention.

### EXAMPLES

Manufacturing a diclofenac sodium releasing bioabsorbable PLGA 80/20 rod.

In this experiment we manufactured diclofenac sodium releasing bioabsorbable rods that had sufficient mechanical properties to be used as fixation devices in repare of bone and cartilage.

### Materials

As a matrix polymer a copolymer of lactide and glycolide (PLGA 80/20) was used. The lactide glycolide molar ratio of the polymer, informed by the manufacturer, was 78 to 22 respectively. The material was obtained from Purac Biochem (Gorinchem, Holland).

As an active agent we used a non-steroidal anti-inflammatory drug (NSAID), diclofenac sodium (DS). DS was purchased in powder form from Sigma-Aldrich (Espoo, Finland).

The choice of DS was based on the following factors:
1. It is one of the most potent NSAIDs for treatment of arthritis disorders.
2. It has high melting point (Tₘ) which allows processing at high temperatures.
3. Reasonably high Cox-2 selectivity for a traditional NSAID. This lowers the risks of gastrointestinal side effects
4. Clinically one of the most effective NSAIDs for treatment of both inflammation and pain.
5. Promising results of earlier experiments of other research groups in combining DS and PLGA.
6. Very widely used drug worldwide for treatment of arthritic disorders.

However, the invention is not limited to only the above-mentioned anti-inflammatory agent, but other such agents being solid at the processing temperatures can be used. Statins can be used in analogical manner and similar requirements are set on them.

### Methods

### Drying and mixing the raw materials.

Raw materials were first dried in vacuum oven for over 24 hours. After drying the DS powder and polymer granules were mixed together using electrical grinder (Retsch Grindomix GM200). Three different DS contents were experimented in two compounding trials. In the first experiment (E-1) 8 wt-% of DS was mixed. In the second experiment (E-2) 4 and 2 wt-% of DS was used. The mixing parameters were adjusted in a way that attachment of the drug particles to the surface of the polymer granules would we as good as possible. The mixed raw materials were again put to vacuum for 24 hours.

### Compounding

A laboratory twin-screw extruder was used to produce DS containing PLGA 80/20 rods. Raw materials were fed to the extruder using single-screw feeding unit. Pressurized air and water-cooled cooling plate were used for billet cooling. In E-1 billet drawing was performed with a manually controlled cooling plate. In E-2 billet drawing and optimization of dimensions was performed with an automatic laser measurement unit combined with a automatic drawing unit. Dies of ø3 mm and ø7 mm were used in E-1 and E-2 respectively to produce rods with diameters ~3.1 mm and ∼6.6 mm.

### Self-Reinforcing

Manufactured rods were self-reinforced (SR) using die-drawing. When part of the microstructure of the polymer is transformed into oriented form by SR, the mechanical properties (strength, modulus and toughness) of these materials increase significantly. Rods were kept inside a heated cylinder and drawn through a heated die using a specially designed self-reinforcing machine. Temperatures of the cylinder and die were both 87 °C. For E-1 rods ø1.25 mm drawing die was used. For E-2 rods ø3.3 and 3.4 Teflon® coated drawing dies were used. Drawing speeds were 23-24 mm/min E-1 rods and 16-18 mm/min for E-2 rods. SR-E-1 rods were 1.06-1.16 mm, and SR-E-2 rods 3.06 - 3.36 mm in diameter. SR-rods were also gamma-sterilized with 25 kGy dose.

### Analysis

DS containing rods were analyzed for mechanical properties (shear- and bending strength, and Young's modulus), viscosity, microstructure (scanning electron microscopy) and drug release properties. Plain PLGA 80/20 rods were used as control. In vitro model was used to analyze changes in viscosity and mechanical properties. In the in vitro-model, the rods were incubated in phosphate buffer solution (KH2PO4 + NaOH , pH 7,4 ± 0,02) and their properties were measured at predesigned intervals. Drug release was measured as a concentration of the drug in the PBS using UV-spectrophotometry. Rods were analyzed at different manufacturing stages - as compounded (CO-) self reinforced (SR-) and self-reinforced and gamma-sterilized (sSR-).

### Results

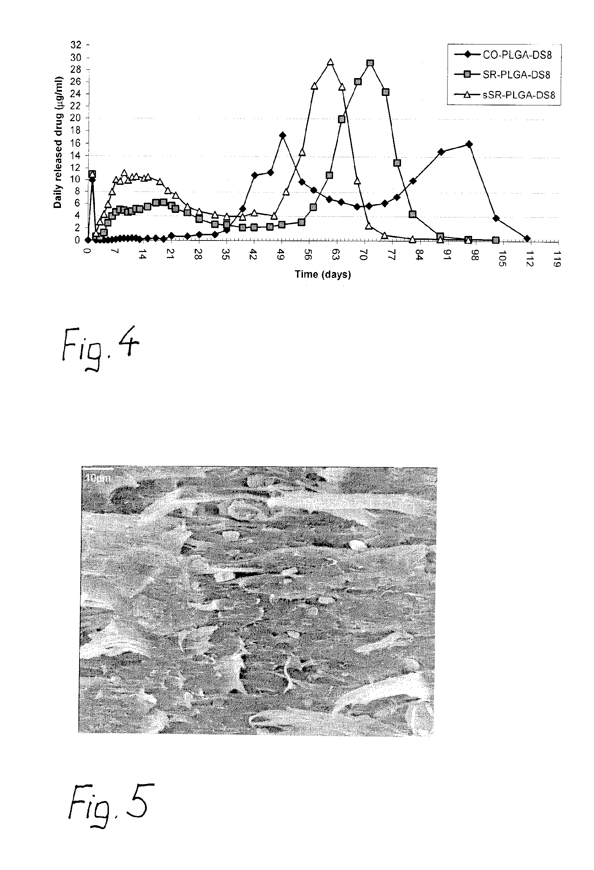

DS containing PLGA 80/20 rods had sufficient mechanical properties to be used as fixation devices in fixation of bone and cartilage. The rods released the drug in 82 - 168 days depending on rods dimensions, manufacturing techniques and gamma-sterilization. SR and gamma-sterilization both increased the amount of release during first 4-5 weeks. Location of the burst peak of SR rods was also shifted to earlier by the result of gamma-sterilization (Fig. 4). The rods containing 4 wt-% of DS showed the same behaviour. For analysis of microstructure by SEM showed that drug was dispersed all around in the matrix but had tendency to flocculate.

The mechanical values are shown in the following Table 1 and the SEM-image of the oriented structure containing the DS particles, in 1000X magnification, in Fig. 5.

**Table 1.**

| Mean values and standard deviation (STD) of shear and bending strength in megapascals (MPa) as well as Young's modulus in bending (YM) in gigapascals (GPa) of compounded (CO-), self-reinforced (SR) and gamma-sterilized self-reinforced (sSR-) rods made of PLGA 80/20 that contain diclofenac sodium (DS) 8wt.-%, compared to pure SR-PLGA rods. | | | | | | |
|---|---|---|---|---|---|---|
| **Rod composition** | **Bending Strength (MPa)** | **STD** | **Modulus (GPa)** | **STD** | **Shear Strength (MPa)** | **STD** |
| **SR-PLGA 100%** | 320,8 | 7,8 | 20,17 | 0,77 | 107,7 | 1,7 |
| **sSR-PLGA 100%** | 285,1 | 7,4 | 19,93 | 0,17 | 109,4 | 1,1 |
| **CO-PLGA 92% + DS 8%** | 86,85 | 1,66 | 1,302 | 0,05 | 55,14 | 2,11 |
| **SR-PLGA 92% + DS 8%** | 243,7 | 46,4 | 16,11 | 2,75 | 88,03 | 1,1 |
| **sSR-PLGA 92 %+ DS 8%** | 271,2 | 21 | 16,98 | 1,07 | 93,37 | 5,08 |

### DICLOFENAC EXAMPLE (MONOFILAMENTS)

### Example about a monofilament

### Compounding and melt-spinning

Commercial PuraSorb®PLG (Purac Biochem bv., Gorinchem, Netherlands) was used as basic polymer material. Diclofenac was compounded into PLGA 80L/20G matrix with a small twin-screw extruder. The loading of diclofenac was 4 wt-%. Reference PLGA 80L/20G and diclofenac containing (DS) PLGA 80L/20G monofilaments were melt-spun by small laboratory extruder. The filaments were orientated in-line during melt-spinning. Drawing ratio was 4.8.

### Tensile test of monofilaments

The tensile test for non-sterile monofilaments was done with Instron 4411 universal testing machine (Instron Ltd., Hiwh Wycombe, England). The test was performed using pneumatic jaws in distance of 50 mm. The tensile speed was 30 mm/min. The test was performed using the force cell of 500 N. Five parallel samples were taken about 50 cm distance from each other.

### Release of diclofenac in vitro

Release on diclofenac *in vitro* has been studied with the sample:

| Sample | Sample [mg] | Buffer [ml] |
|---|---|---|
| PLGA 80L/20G-DS4 | 200 | 10 |

In all parallel series buffer was (KH₂PO₄ and NaOH) adjusted in pH 7,4 ± 0,02. The samples were incubated at 37°C and buffer was replaced after specific time periods. From replaced buffer released quantity of diclofenac from monofilaments was detected by spectrophotometer (UNICAM UV 540, Thermo Spectronic, Cambridge, UK). The maximum absorption was measured (λ = 275 nm) from the samples and the quantity of released analgesic was calculated according to the Beer-Lambert law. The number of parallel samples was two.

### RESULTS

### Mechanical properties and diclofenac release

The results of preliminary tensile test of nonsterile DS-monofilament are presented in table 2.

**Table 2.**

| The results of tensile test for PLGA 80L/20G-DS4, the sample diameters are 1: 0,363 mm, 2: 0,412 mm, 3: 0,400 mm, 4: 0,382 mm and 5: 0,462 mm. | | | | | | | | |
|---|---|---|---|---|---|---|---|---|
| Specimen number | Load at Max. Load [N] | Stress at Max. Load [MPa] | Strain at Max. Load [%] | Displacement at Max. Load [mm] | Young's Modulus [MPa] | Load at thresh Yield [MPa] | Stress at Yield [MPa] | Display at [nm] |
| 1 | 7.22 | 69.77 | 72.60 | 36.40 | 3344.90 | | 56.8 | |
| 2 | 6.03 | 45.21 | 52.03 | 26.06 | 2118.11 | | 22.8 | |
| 3 | 7.40 | 58.86 | 96.33 | 48.25 | 2568.14 | | 28.6 | |
| 4 | 7.52 | 65.59 | 85.20 | 42.67 | 2839.16 | | 36.3 | |
| 5 | 9.30 | 55.49 | 123.70 | 62.09 | 1864.74 | | 28.7 | |
| Mean | 7.49 | 58.98 | 85.97 | 43.10 | 2547.01 | | 34.7 | |
| S.D | 1.17 | 9.51 | 26.75 | 13.44 | 585.64 | | 13.3 | |

The results of PLGA-reference filament are presented in table 3 below.

**Table 3.**

| The results of tensile test of reference PLGA 80L/20G filaments, the sample diameters are 1: 0,26 mm, 2: 0,25 mm, 3: 0,26 mm, 4: 0,25 mm and 5: 0,25 mm. | | | | | | | | |
|---|---|---|---|---|---|---|---|---|
| Specimen number | Load at Max. Load [N] | Stress at Max. Load [MPa] | Strain at Max. Load [%] | Displacement at Max. Load [mm] | Modulus (Aut Youg) [MPa] | Load at Yield [MPa] | Stress at Yield [Mpa] | Display at [nm] |
| 1 | 16.81 | 316.62 | 24.16 | 12.05 | 5146.82 | | 316.5 | |
| 2 | 16.08 | 327.58 | 26.46 | 13.25 | 5613.80 | | 327.6 | |
| 3 | 17.70 | 333.38 | 27.99 | 14.01 | 5370.88 | | 332.2 | |
| 4 | 16.86 | 343.47 | 25.46 | 12.76 | 5715.23 | | 341.8 | |
| 5 | 18.23 | 371.38 | 28.20 | 14.12 | 5966.01 | | - | |
| Mean | 17.14 | 338.48 | 26.45 | 13.24 | 5562.55 | | 329.53 | |
| S.D | 0.84 | 20.80 | 1.71 | 0.87 | 315.55 | | 10.50 | |

The release of diclofenac is shown below:

| Time [h] | Abs. 1 | Abs. 2 |
|---|---|---|
| 6 | 0.6593 | 0.639 |
| 18 | 0.038 | 0.0615 |
| 48 | 0.0176 | 0.0355 |
| 72 | 0.0169 | 0.03 |

### Conclusions

The addition of diclofenac increases tensile elongation of monofilaments and decreases Young's modulus of monofilaments. Diclofenac containing monofilaments are more elastic compared to to neat reference PLGA 80L/20G monofilaments.

### Possible applications in surgery

In a surcical method related to bone tissue in a mammal, e.g. human patient, the implant can be used together with demineralized bone matrix. The implant can also be used together with bone graft. The implant can also be used with other bone graft substitutes.

The surgical method provides enhancing the healing of a wound, injury, or defect in the bone in a mammal, e.g. human patient, an it comprises administering at the site a surgical implant or surgical device using open surgery or minimal access surgery, or combined minimal access and open surgery.

The surgical implant or surgical device can be in the form of a bone to bone, bone to cartilage, or soft tissue to bone or soft tissue- to- soft tissue fixation device, a device for guided tissue regeneration, such as scaffold, or a device for tissue augmentation.

The above-mentioned fixation device used in the method can be in the form of a pin, screw, plate, tack, intramedullary nail, bolt, suture anchor, arrow or tissue anchor, interference screw or wedge.

Further, the surgical implant or the fixation device can be a suture, sheet, membrane, stent, filament, fiber, felt, or fabric.

## Claims

1. A multifunctional biodegradable composite that has:
a) bioabsorbable polymer matrix phase (M)
b) bioabsorbable reinforcing element (R), and
c) bioactive tissue/cell reaction modifying agent (TRMA) dispersed in said bioabsorbable matrix phase and selected from the group consisting of anti-inflammatory drugs and statins.

2. The composite of claim 1, wherein the bioabsorbable polymer is a poly(alpha-hydroxy acid).

3. The composite of claim 2, wherein the poly(alpha-hydroxy acid) is a copolymer of lactide and glycolide.

4. The composite of claim 3, wherein the poly(alpha-hydroxy acid) is a copolymer of lactide and glycolide in the molar percentage ratio range between 75 to 25 and 85 to 15, respectively.

5. The composite of any of the preceding claims 1 to 4, wherein the reinforcing element is also made of a biodegradable polymer

6. The composite of claim 5, wherein the reinforcing element is made of the same polymer as the matrix.

7. The composite of claim 6, wherein the composite is self-reinforced and/or oriented.

8. The composite of any of the preceding claims, wherein the biodegradable reinforcing element is a plurality of fibers, fibrils, oriented polymer chains, or a combination of thereof.

9. The composite of any of the preceding claims, wherein it is arranged to stimulate and/or enhance bone healing.

10. The composite of any of the preceding claims, wherein the anti-inflammatory drug is selected from a group consisting of nonsteroidal anti-inflammatory drugs (NSAIDs).

11. The composite of claim 10, wherein the nonsteroidal anti-inflammatory drug is diclofenac acid or its salt(s).

12. The composite of any of the preceding claims, wherein the statin is simvastatin.

13. The composite of any of the preceding claims, wherein the bioactive agent is used in the percentage of 0.1 to 10 % by weight, on the basis of total weight of the composite.

14. The composite of any of the preceding claims, wherein the combination of the bioabsorbable polymer matrix phase and the reinforcing element comprises cavities or pockets containing particles of the bioactive agent.

15. The composite of claim 14, wherein the cavities or pockets contain voids around the particles of the bioactive agent.

16. The composite of claim 14 or 15, wherein the cavities or pockets are created by mechanical deformation of the bioabsorbable polymer matrix phase.

17. The composite of claim 16, wherein the cavities or pockets are created by orientation of the bioabsorbable polymer matrix phase.

18. The composite of any of claims 1 to 17, wherein the additional fourth component is added.

19. The composite of claim 18, wherein the fourth component is a bioceramic particle or fibre such as calcium phosphate or bioactive glass.

20. A surgical implant capable of acting as a drug-delivery implant for controlled release of an anti-inflammatory drug or statin and comprising a composite of any of the preceding claims.

21. The surgical implant of claim 20, wherein it is in the form of a bone to bone, bone to cartilage, or soft tissue to bone or soft tissue- to- soft tissue fixation device, a device for guided tissue regeneration, such as scaffold, or a device for tissue augmentation.

22. The surgical implant of claim 21, wherein the fixation device is in the form of a pin, screw, plate, tack, intramedullary nail, bolt, suture anchor, arrow or tissue anchor, interference screw or wedge.

23. The surgical implant of claim 21 or 22, wherein it is a suture, sheet, membrane, stent, filament, fiber, felt, or fabric.

24. The surgical implant of any of the preceding claims 20 to 23, wherein the drug delivery implant is arranged to release the anti-inflammatory drug or statin from said composite in therapeutic concentrations for a period of at least 24 hours after implantation up to 4 weeks or more in *in vivo* conditions once implanted in a mammalian body.

25. The surgical implant of any of the preceding claims 20 to 24, wherein the implant is packaged and sterilized.

26. A method for manufacturing a composite of any of the preceding claims 1 to 19, comprising the steps of
- forming a mixture of a polymer melt and anti-inflammatory drug or statin particles dispersed into the polymer melt,
- forming a longitudinal item from the polymer melt-drug dispersion mixture by pressing the polymer, melt-drug dispersion mixture through a die,
- cooling of the aforesaid mixture to solidify it to a solid item,
- deforming mechanically the solidified item at a temperature T>Tg, where Tg is the glass transition temperature of the polymer, to orient it longitudinally.

27. The method of claim 26, wherein the anti-inflammatory drug is selected from the group consisting of non-steroidal anti-inflammatory drugs (NSAIDs).

28. The method of claim 26 or 27, wherein cavities or pockets containing particles of the anti-inflammatory drug or statin are formed during the orientation.

29. The method of claim 26, 27 or 28, wherein the composite is formed into or made part of a surgical implant, the implant is packaged, and sterilized before the packaging or after the packaging.

30. The method of claim 29, wherein the sterilization is performed by gamma-radiation.

31. The method of claim 29, wherein the sterilization is performed by ethylene-oxide sterilization (ETO).

32. The method of claim 29, 30 or 31, wherein the composite is formed into or made part of a scaffold, stent, fastener, rod or pin, screw, tack, plate, expansion plug, staple, repair patch, filling/bulking material, or membrane.

33. The use of a multifunctional biodegradable composite according to any of claims 1 to 19 to manufacture a pharmaceutical drug-delivery surical implant for treating or preventing inflammation or pain and/or for enhancing hard tissue growth or regeneration, by applying said implant to hard tissue or near hard tissue.
